# EUROPEAN PATENT APPLICATION

(11) **EP 4 721 788 A1**
(43) Date of publication of application: **08.04.2026**
(21) Application number: 23939452.1
(22) Date of filing: 26.05.2023
(51) Int. Cl.: A61M 15/00, A61M 13/00

(54) **SUCTION DEVICE AND SUCTION SYSTEM**

(71) Applicant: Japan Tobacco Inc., Tokyo 105-6927 (JP)
(72) Inventor: FUJITA, Ryoji, Tokyo 130-8603 (JP); HODONO, Hiroshi, Tokyo 130-8603 (JP); WAKAMATSU, Miki, Tokyo 130-8603 (JP); NAKANO, Takuma, Tokyo 130-8603 (JP); SADAKARI, Kei, Tokyo 130-8603 (JP); TAKANABE, Yurina, Tokyo 130-8603 (JP)
(74) Representative: Hoffmann Eitle
(86) International application number: PCT/JP2023/019613
(87) International publication number: WO 2024/246967

(57) **Abstract**

A suction device comprising: a housing; a storage part capable of storing a consumable material in the housing; a perforating part for opening a hole on a surface of the consumable material; and a detection part for enabling the detection of whether the hole was correctly opened by the perforating part.

## Description

### TECHNICAL FIELD

The present invention relates to an inhalation device and an inhalation system.

### BACKGROUND ART

There are known inhalation devices in which a hole is formed in a consumable material containing a powder, etc., in order to release the powder, etc. from the consumable material. In the inhalation system of PTL 1, a piercing element comprises only a single shaft extending from a fixed end to a tip end along a long axis, the shaft being configured to pierce a capsule inside an inhalation article.

### CITATION LIST

### PATENT LITERATURE

PTL 1: WO 2022/123486 A1

### SUMMARY OF INVENTION

### TECHNICAL PROBLEM

The feature of the inhalation system of PTL 1 lies in that the long axis of the piercing portion is offset from the long axis of the capsule and the long axis of a sleeve of the inhalation system, so that the piercing portion improves capsule reliability. However, the piercing portion does not always correctly pierce the surface of the consumable material.

In light of the above, one objective of the present invention lies in providing an inhalation device and an inhalation system enabling a user or the inhalation device to respond to a situation in which a hole has not been correctly formed in the surface of a consumable material by a piercing portion.

### SOLUTION TO PROBLEM

A first aspect provides an inhalation device. This inhalation device comprises: a housing; an accommodating portion enabling a consumable material to be accommodated inside the housing; a piercing portion for forming a hole in a surface of the consumable material; and a detection unit for enabling detection of whether or not the hole has been correctly formed by the piercing portion.

According to the first aspect, it is possible to provide an inhalation device enabling a user, etc. to respond to a situation in which a hole has not been correctly formed in the surface of the consumable material by the piercing portion.

The essential point of the second aspect, which is in accordance with the first aspect, is that the detection unit is configured to detect a pressure applied to the piercing portion.

According to the second aspect, the inhalation device is capable of obtaining information relating to what nature of contact the piercing portion has made with the consumable material, etc., and is capable of detecting with greater certainty whether or not a hole has been correctly formed in the consumable material.

The essential point of a third aspect, which is in accordance with the second aspect, is that the detection unit is a pressure sensor.

According to the third aspect, the inhalation device is capable of detecting more accurately whether or not a hole has been correctly formed in the consumable material, by utilizing measured pressure data.

The essential point of a fourth aspect, which is in accordance with the third aspect, is that the piercing portion is a rod-shaped or needle-shaped member extending in a longitudinal direction, and the pressure sensor is disposed on the opposite side along the longitudinal direction to the side on which the consumable material is arranged

According to the fourth aspect, the pressure sensor readily detects a force in a movement direction when the piercing portion and the consumable material come closer together, and the inhalation device is capable of detecting even more accurately whether or not a hole has been correctly formed in the consumable material.

The essential point of a fifth aspect, which is in accordance with the third or fourth aspect, is that a control unit is further provided to determine whether or not the hole has been correctly formed in the consumable material by the piercing portion.

According to the fifth aspect, the inhalation device is capable of detecting more accurately whether or not a hole has been correctly formed in the consumable material by analyzing, etc. the pressure data.

The essential point of a sixth aspect, which is in accordance with the fifth aspect, is that the control unit comprises a memory unit for storing a pressure value from when the piercing portion formed a hole in the surface of the consumable material, and makes the abovementioned determination based on the pressure value and output from the pressure sensor.

According to the sixth aspect, the inhalation device is capable of detecting more accurately whether or not a hole has been correctly formed in the consumable material, by utilizing data, etc. obtained in the past.

The essential point of the seventh aspect, which is in accordance with the fifth or sixth aspect, is that, based on whether or not the pressure obtained from the pressure sensor is rising, the control unit determines at least one of: whether or not to continue measurement by the pressure sensor, and whether or not to record the measured pressure.

According to the seventh aspect, it is possible to reduce power consumption and to cause the memory unit to store pressure data efficiently.

The essential point of an eighth aspect, which is in accordance with the fifth aspect, is that the piercing portion is a rod-shaped or needle-shaped member extending in a longitudinal direction, and the pressure sensor is configured to detect a pressure on the piercing portion in a direction intersecting the longitudinal direction.

According to the eighth aspect, it is possible to detect bending or flexing of the piercing portion.

The essential point of a ninth aspect, which is in accordance with any of the fifth to eighth aspects, is that the control unit is configured to notify a user of information relating to the abovementioned determination by means of at least one of sound, light, a screen, and vibration.

According to the ninth aspect, information relating to whether or not a hole has been correctly formed in the consumable material can be transmitted to the user more reliably.

The essential point of a 10th aspect, which is in accordance with any of the fifth to ninth aspects, is that, based on the abovementioned determination, the control unit is configured to switch between an inhalation-possible state and an inhalation-impossible state of the inhalation device.

According to the 10th aspect, it is possible to prevent the user from trying to inhale when a hole has not been correctly formed by the piercing portion, but this leading to adverse effects such as the user being unable to inhale suitably, or the inhalation device being damaged.

The essential point of an 11th aspect, which is in accordance with the 10th aspect, is that the control unit is configured to perform the abovementioned switch by controlling opening/closing of a flow path for introducing air into the accommodating portion.

According to the 11th aspect, it is possible to more reliably prevent the user from trying to inhale, but this leading to adverse effects such as the user being unable to inhale suitably, or the inhalation device being damaged.

The essential point of a 12th aspect, which is in accordance with any of the fifth to 11th aspects, is that the control unit is configured to switch the pressure sensor between a detection-possible state and a detection-impossible state according to a position of a shutter disposed at an introduction port for introducing air into the accommodating portion.

According to the 12th aspect, measurement by the pressure sensor can be stopped when it is unlikely that the consumable material is inserted in the accommodating portion, and it is possible to reduce power consumption.

The essential point of a 13th aspect, which is in accordance with the first aspect, is that the detection unit is a mechanical structure which is configured so that, when the consumable material has been inserted into the accommodating portion but the hole has not been formed by the piercing portion, a portion of the piercing portion is pushed out, to a position where it is visible from outside of the inhalation device, through a weakened portion or a through-hole formed in an inner wall of the accommodating portion.

According to the 13th aspect, when a hole has not been correctly formed in the surface of the consumable material, it is possible to detect that the hole has not been correctly formed, without electrical power necessarily being required, therefore enabling a user, etc. to respond to the situation.

The essential point of the 14th aspect, which is in accordance with any of the first to 13th aspects, is that the consumable material comprises a flavor powder, and the inhalation device is a flavor inhaler for inhaling a flavor.

According to the 14th aspect, the inhalation device can provide a user with the opportunity to enjoy the flavor.

A 15th aspect provides an inhalation system. This inhalation system comprises: the inhalation device according to any of the first to 14th aspects; and a consumable material which can be accommodated in the inhalation device.

According to the 15th aspect, it is possible to provide an inhalation system enabling a user, etc. to respond to a situation in which a hole has not been correctly formed in the surface of the consumable material by the piercing portion.

### BRIEF DESCRIPTION OF DRAWINGS

Fig. 1 is an outline diagram showing an inhalation system according to a first embodiment.
Fig. 2 is a view in cross section schematically showing an inhalation article according to the first embodiment.
Fig. 3A is a side view schematically showing a consumable material according to the first embodiment.
Fig. 3B is a view in cross section schematically showing particles contained in the consumable material.
Fig. 4 is a view in cross section schematically showing an inhalation device according to the first embodiment.
Fig. 5 is a view in cross section of the inhalation system, schematically showing piercing performed by the piercing portion.
Fig. 6 is a graph showing a relationship between time and pressure at the time of piercing by the piercing portion.
Fig. 7 is a view in cross section schematically showing an insertion sensor according to the first embodiment.
Fig. 8 is a flowchart showing a flow of control by the control unit in the first embodiment.
Fig. 9 is a view in cross section schematically showing an inhalation device according to a second embodiment.
Fig. 10 is an outline diagram to illustrate detection of whether or not a hole has been correctly formed, in the second embodiment.
Fig. 11 is a side view schematically showing a piercing portion according to a third embodiment.
Fig. 12 is an outline diagram to illustrate detection of whether or not a hole has been correctly formed, in the third embodiment.

### DESCRIPTION OF EMBODIMENTS

Embodiments of the present invention will be described below with reference to the drawings. In the drawings described below, identical or corresponding components are assigned the same reference numbers and duplicate descriptions will not be given. Dimensions of parts in the drawings below are altered, as appropriate, for ease of understanding.

### First embodiment

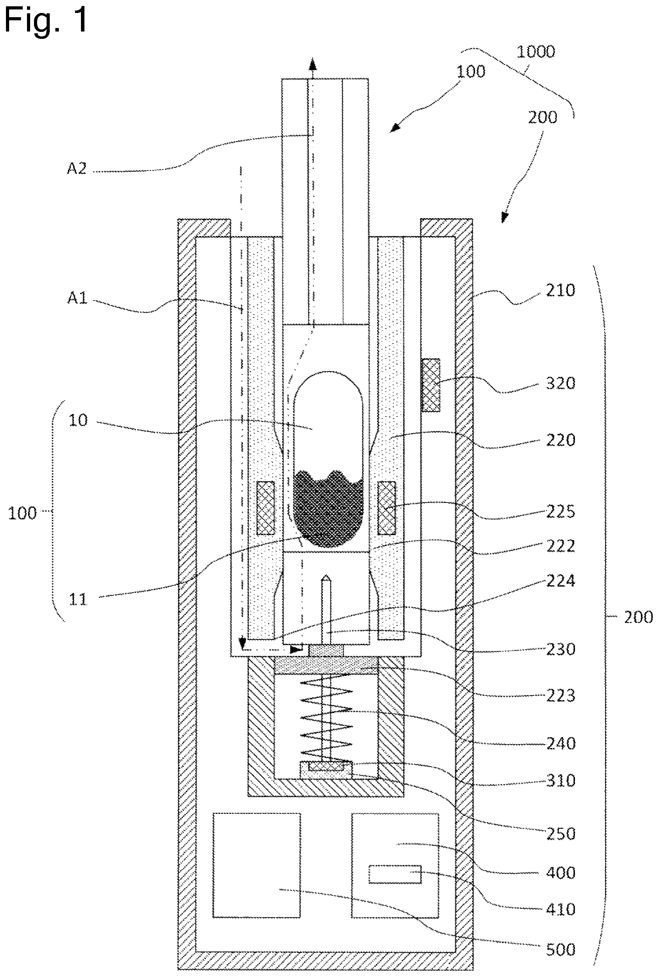

Fig. 1 shows an inhalation system 1000 according to the first embodiment. As shown in fig. 1, the inhalation system 1000 comprises: an inhalation article 100 having a consumable material 10 containing an inhalable powder 11; and an inhalation device 200 which comprises a piercing portion 230 for piercing the consumable material 10, and releases the powder 11 so that it can be inhaled by a user. The inhalation system 1000 is configured so that the piercing portion 230 forms a hole in a surface of the consumable material 10 which has been inserted into the inhalation device 200, and the powder 11 released from the hole is released to outside of the inhalation device 200. Air passes through a first air flow path A1 formed in the inhalation device 200, is then introduced into a second air flow path A2 formed in the inhalation article 100, and is released to outside of the inhalation system 1000 while carrying the powder 11. In this way, the inhalation device 200 and the inhalation system 1000 function as a powder inhaler and a powder inhalation system for allowing a user to inhale the powder 11.

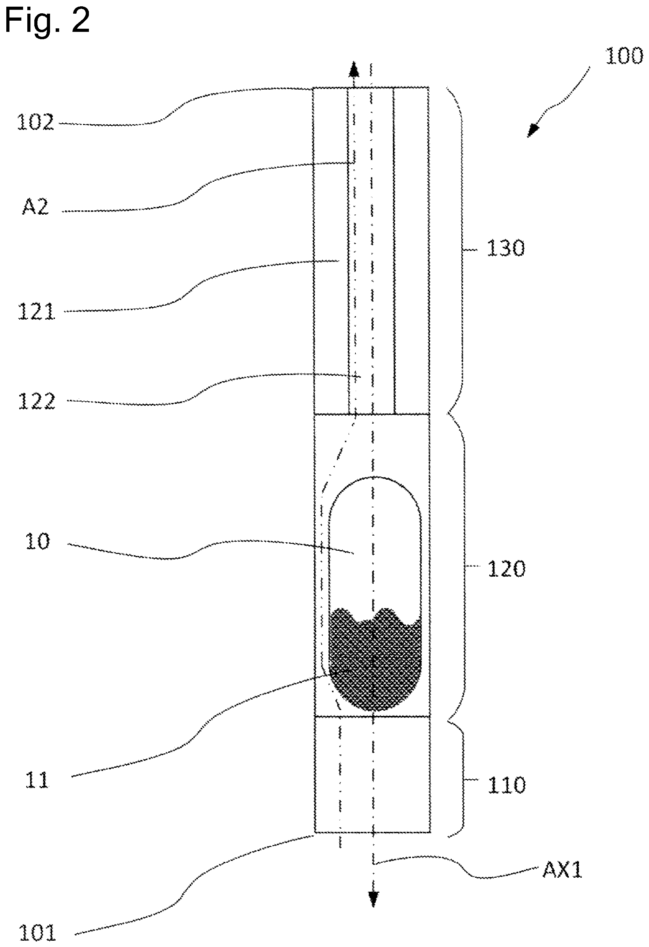

Fig. 2 is a view in cross section schematically showing the inhalation article 100. The inhalation article 100 is configured so that it can be accommodated in the inhalation device 200. The inhalation article 100 comprises a sealing segment 110, a powder-containing segment 120, and a mouthpiece segment 130. The sealing segment 110, the powder-containing segment 120, and the mouthpiece segment 130 are arranged along an insertion direction, which is a direction in which the inhalation article 100 is inserted into the inhalation device 200. In the example depicted, the insertion direction is a direction of extension of a long axis AX1 of the inhalation article 100. The inhalation article 100 comprises: a first end 101 which is inserted into the inhalation device 200, and a second end 102 formed on the opposite side to the first end 101.

The sealing segment 110 is a segment for sealing the consumable material 10 inside the inhalation article 100, and is arranged on the first end 101 side of the powder-containing segment 120. The sealing segment 110 is configured so that it can be penetrated by a piercing portion 230. The configuration is preferably such that the sealing segment 110 is penetrated by the piercing portion 230 in the insertion direction when the inhalation article 100 is inserted into the inhalation device 200. The sealing segment 110 is formed with an air flow path enabling communication between the first end 101 side and the second end 102 side thereof, and this air flow path forms part of the second air flow path A2.

The powder-containing segment 120 is a segment in which the consumable material 10 is arranged. The consumable material 10 is a container such as a capsule which internally contains the powder 11. A surface of the consumable material 10 is configured so that it can be pierced by the piercing portion 230. The configuration is preferably such that the surface of the consumable material 10 is penetrated by the piercing portion 230 in the insertion direction when the inhalation article 100 is inserted into the inhalation device 200. The powder-containing segment 120 is formed with an air flow path enabling communication between the first end 101 side and the second end 102 side thereof, and this air flow path forms part of the second air flow path A2. When a hole is formed in the surface of the consumable material 10 by the piercing portion 230, the powder 11 is released from this hole into a space where the consumable material 10 is disposed in the powder-containing segment 120. The released powder 11 is introduced into the mouthpiece segment 130 together with air while being suitably agitated by the flow of air.

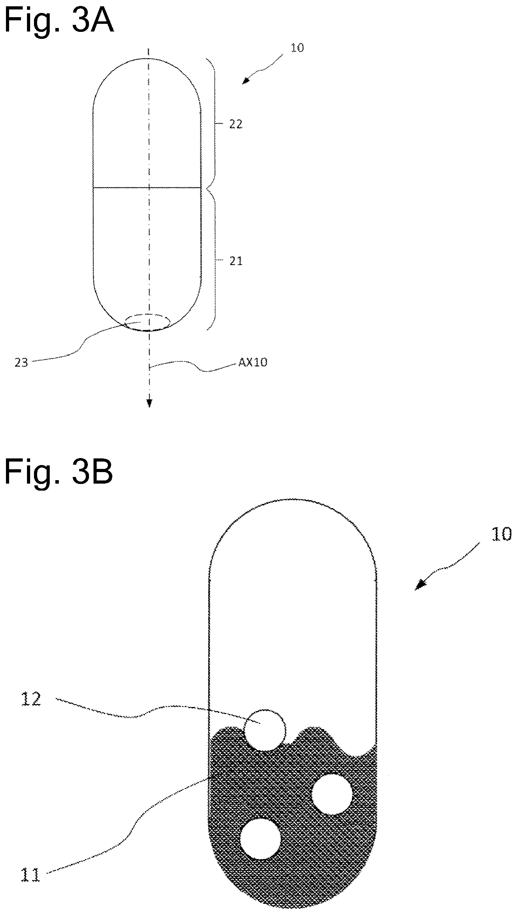

Fig. 3A is a side view schematically showing the consumable material 10. In the example depicted, the consumable material 10 is a capsule. The consumable material 10 comprises a first part 21 and a second part 22. The first part 21 is arranged further to the first end 101 side of the inhalation article 100 than the second part 22. The first part 21 and the second part 22 are preferably aligned on a long axis AX10 of the consumable material 10.

A consumable material weakened portion 23 is formed at the end portion of the first part 21 on the opposite side to the second part 22. The consumable material weakened portion 23 is formed by a material which is weaker than the piercing portion 230, and allows the surface of the consumable material 10 to be pierced more easily by the piercing portion 230. Furthermore, the correct piercing position can be ensured by making the end portion of the consumable material 10 facing the piercing portion 230 weaker so that it can be pierced, and by making other parts harder. From this perspective, the consumable material weakened portion 23 is preferably formed so as to include the long axis AX10 of the consumable material 10. The consumable material 10 is preferably arranged in the inhalation article 100 so that the long axis AX10 of the consumable material 10 and a long axis AX100 of the inhalation article 100 are roughly aligned. The consumable material weakened portion 23 is preferably arranged so as to face the piercing portion 230 in the direction of the long axis AX10 when the inhalation article 100 is accommodated in the inhalation device 200. It should be noted that the consumable material weakened portion 23 need not be formed, provided that the consumable material 10 can be pierced by the piercing portion 230.

There is no particular limitation as to the powder 11, provided that it is a substance which can be inhaled by the user. The powder 11 may be a flavor powder containing a flavor, or a medication such as a drug. When the powder 11 is a flavor powder, there is no particular limitation as to the type thereof provided that it contains a flavor and can be inhaled. The powder 11 may comprise nicotine, various types of flavoring materials such as menthol, sugars, or amino acids. The powder 11 may be produced by means of spray drying. Sugar alcohols and amino acids may be used as excipients of the powder 11. Specifically, excipients may be selected from mannitol, trehalose, leucine, alanine, valine, isoleucine, methionine, phenylalanine, tyrosine, and tryptophan, which may also be used in combination. When the powder 11 is a flavor powder, the inhalation device 200 is constructed as a flavor inhaler for inhaling the flavor, and the inhalation article 100 is constructed as a flavor-generating article. As a result, the inhalation device 200 can provide the user with the opportunity to enjoy the flavor.

As shown in fig. 3B, the consumable material 10 preferably contains particles 12 for crushing the powder 11 contained in the consumable material 10. Fig. 3B is a view in cross section schematically showing the particles 12. There is no particular limitation as to the shape and material of the particles 12, provided that they promote dispersion of the agglomerated powder 11 by means of vibration, which is produced by means of a vibration generating unit 225 to be described later. The particles 12 preferably have a maximum diameter which is larger than the maximum diameter of the powder 11. The particles 12 preferably have a minimum diameter which is larger than the minimum diameter of the hole formed by the piercing portion 230, in order to limit adverse effects such as the particles 12 being released to outside of the consumable material 10 and damaging the inhalation article 100, or the particles 12 being inhaled. It should be noted that the consumable material 10 need not contain the particles 12.

As shown in fig. 2, the mouthpiece segment 130 is arranged on the second end 102 side of the powder-containing segment 120 and is a segment which functions as a mouthpiece. The mouthpiece segment 130 preferably includes a tubular member 121 in which a hollow portion 122 is formed, as in the example depicted. The tubular member 121 is adjusted to a hardness so that the user can easily hold it in their mouth. The mouthpiece segment 130 is formed with an air flow path enabling communication between the first end 101 side and the second end 102 side thereof, and this air flow path forms part of the second air flow path A2. In the example depicted, the hollow portion 122 forms part of the second air flow path A2. The powder 11 and air introduced from the powder-containing segment 120 are released into the user's oral cavity through the second air flow path A2 inside the mouthpiece segment 130.

In the example depicted, the inhalation article 100 has a columnar shape from the perspective of ease of holding by the user. The inhalation article 100 is preferably cylindrical. However, there is no particular limitation as to the shape of the inhalation article 100 provided that the consumable material 10 can be pierced by the piercing portion 230 and the powder 11 can be inhaled by the user. The inhalation article 100 may have a columnar cross section with any polygonal shape, or may have a flat shape. Furthermore, the inhalation article 100 may have any number of segments, or may be lacking at least one of the segments mentioned above.

Moreover, the inhalation article 100 may comprise only the consumable material 10. In this case, the powder 11 released from the hole formed by the piercing portion 230 is released to outside of the inhalation device 200 through a space in the accommodating portion 220 outside the consumable material 10. In this case, when the consumable material weakened portion 23 is formed only at one end of the consumable material 10, there is a risk of the user erroneously inserting the consumable material 10 into the accommodating portion 220 in such a way that the consumable material weakened portion 23 does not face the piercing portion 230, but this can be detected by a detection unit 310 in the present embodiment. Furthermore, in this case, the inhalation device 200 may further comprise a mouthpiece portion functioning as a mouthpiece. The mouthpiece portion may be detachably fitted to the inhalation device 200. For example, the inhalation system 1000 may be configured so that the consumable material 10 such as a capsule is loaded in the inhalation device 200, then the mouthpiece portion is engaged with the inhalation device 200 to allow inhalation.

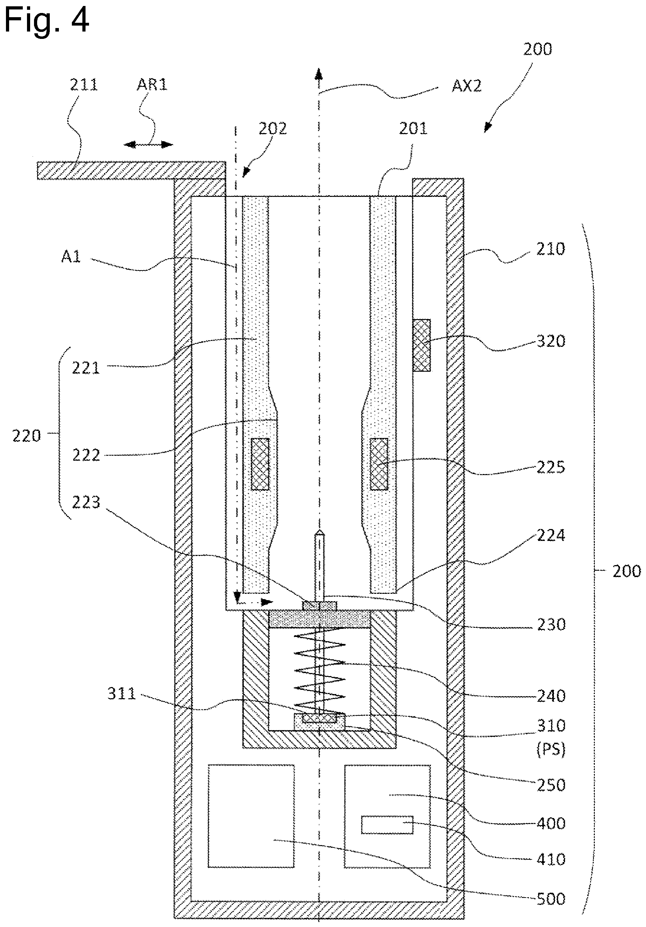

Fig. 4 is a view in cross section schematically showing the inhalation device 200. The inhalation device 200 comprises a housing 210 and an accommodating portion 220 enabling the consumable material 10 to be accommodated inside the housing 210. The housing 210 is configured to cover the accommodating portion 220 and accommodate the parts constituting the inhalation device 200. The housing 210 is preferably formed by an elastic material such as an elastic resin. By this means, when intense vibration is generated by the vibration generating unit 225, to be described later, it is possible to inhibit an undesirable sensation from being imparted to the user. From this perspective, an elastic member may be disposed between the housing 210 and the accommodating portion 220, and the housing 210 need not comprise an elastic material. As shown in fig. 4, the inhalation device 200 is formed in a columnar shape having, as its axis, a center axis AX2 of the accommodating portion 220, extending in the longitudinal direction. The inhalation device 200 preferably has a columnar shape or a flat shape from the perspective of ease of holding by the user, but there is no particular limitation as to the shape thereof provided that it can accommodate the inhalation article 100 to enable inhalation.

The accommodating portion 220 is configured to accommodate the inhalation article 100 containing the powder 11 for inhalation. The accommodating portion 220 has a tubular portion 221 and a bottom portion 223. A hollow is formed in the tubular portion 221, and the inhalation article 100 is arranged in this hollow. The accommodating portion 220 is formed with an insertion end 201 into which the inhalation article 100 is inserted. An opening is formed at the insertion end 201, and this opening communicates with the hollow in the tubular portion 221. The accommodating portion 220 does not especially need to be heat resistant, etc. so it may be formed by various materials that can be processed into the desired shape, such as a metal, a resin, or a ceramic.

The inhalation device 200 further comprises the vibration generating unit 225. The vibration generating unit 225 is configured to be capable of vibrating the accommodating portion 220. The vibration generating unit 225 comprises a vibration generator such as an eccentric motor. Vibration of the accommodating portion 220 produced by the vibration generating unit 225 is transmitted to the consumable material 10 to cause vibration of the powder 11. The vibration generating unit 225 is preferably arranged at a position overlapping the accommodated consumable material 10 in the longitudinal direction of the inhalation device 200 from the perspective of transmitting vibration more efficiently to the consumable material 10. The vibration generating unit 225 may contact the accommodating portion 220, or may transmit vibration through a member in contact with the accommodating portion 220. In fig. 4, vibration generating units 225 are shown at two locations, but the number of vibration generating units 225 in the inhalation device 200 may be any number of one or more.

If the powder 11 agglomerates in the consumable material 10, this makes the powder 11 less prone to being carried by the flow of air, and it becomes difficult for the powder 11 to be efficiently released to outside of the inhalation device 200. Furthermore, there is a drop in conductance in the second air flow path A2, etc. because of adhesion of the agglomerated powder 11, so efficient release of the powder 11 becomes more difficult from this perspective also. In this embodiment, it is possible to provide an inhalation device 200 having a novel configuration which is capable of inhibiting a drop in the efficiency of releasing the powder 11, even if the powder 11 has agglomerated in the consumable material 10, by virtue of the vibration generating unit 225 which causes vibration of the accommodating portion 220.

As shown in fig. 4, the tubular portion 221 has a gripping portion 222. The gripping portion 222 grips the inhalation article 100 accommodated in the accommodating portion 220. In the example depicted, the gripping portion 222 has a shape protruding into the hollow of the tubular portion 221. From the perspective of gripping the inhalation article 100 more securely, the gripping portion 222 is preferably formed as a single piece with the tubular portion 221 and configured to protrude from the inner wall surface of the tubular portion 221. There is no particular limitation as to the shape of the gripping portion 222, provided that it contacts the inhalation article 100 and transmits vibration thereto, and it may be rib-shaped, etc., for example. The gripping portion 222 preferably does not comprise an elastic member, which would inhibit transmission of the vibration generated by the vibration generating unit 225. The inner diameter of the gripping portion 222 is preferably substantially consistent with the outer diameter of the inhalation article 100. The inner diameter of the gripping portion 222 is preferably slightly smaller than the outer diameter of the inhalation article 100. By this means, the gripping portion 222 can securely abut and grip the inhalation article 100. The inhalation article 100 being gripped by the gripping portion 222 allows the vibration generated by the vibration generating unit 225 to be transmitted more reliably to the consumable material 10.

As shown in fig. 1, the gripping portion 222 is configured to grip the powder-containing segment 120 in a state in which the inhalation article 100 is accommodated in the accommodating portion 220, especially in a state where the consumable material 10 is usably positioned. In the example depicted, the gripping portion 222 grips a portion of the powder-containing segment 120, but may equally grip the entirety of the powder-containing segment 120. By this means, the vibration generated by the vibration generating unit 225 can be more reliably transmitted to the consumable material 10 disposed in the powder-containing segment 120.

The vibration generating unit 225 is preferably provided in the tubular portion 221 from the perspective of transmitting the vibration generated by the vibration generating unit 225 to the consumable material 10 more reliably. The vibration generating unit 225 may be arranged embedded in an inner wall constituting the accommodating portion 220. From the same perspective, the vibration generating unit 225 is more preferably provided in the gripping portion 222. In this case, a part or the entirety of the vibration generating unit 225 should be arranged in the gripping portion 222.

The bottom portion 223 of the accommodating portion 220 is formed on the opposite side of the accommodating portion 220 to the insertion end 201. The bottom portion 223 may function as a stopper for contacting and supporting the first end 101 of the inhalation article 100. The bottom portion 223 preferably comprises a bottom plate, but there is no particular limitation as to the shape thereof. The bottom portion 223 preferably has a projection on the face facing the inhalation article 100, for example. This allows a gap to be formed between the end face of the first end 101 of the inhalation article 100 and the bottom portion 223, as shown in fig. 1, so that air can be introduced more efficiently into the inhalation article 100.

As shown in fig. 4, the accommodating portion 220 has an air introduction port 224. The air introduction port 224 communicates with the outside of the inhalation device 200 through the first air flow path A1. When the user inhales, the air is introduced into the accommodating portion 220 successively through a ventilation port 202, the first air flow path A1, and the air introduction port 224. The first air flow path A1 is an introduction air flow path for introducing air toward the air introduction port 224. The ventilation port 202 is an opening for introducing air into the inhalation device 200, and may be formed at any position on an outer face of the inhalation device 200. In the example depicted, the ventilation port 202 is formed on the same face as the insertion end 201, and a counterflow-type air flow path is formed in the inhalation device 200. The air introduction port 224 is preferably formed further to the bottom portion 223 side than the gripping portion 222, in order to form the second air flow path A2 which passes around the consumable material 10 and carries the powder 11 to the user. In the example depicted, two ventilation ports 202 are provided, one on each side of the center axis AX2, but there is no particular limitation as to the number or position of ventilation ports 202. As shown in fig. 1, air which has been introduced from the air introduction port 224 into the space of the accommodating portion 220 where the inhalation article 100 is accommodated is preferably introduced into the second air flow path A2 of the inhalation article 100 from the end face at the first end 101 of the inhalation article 100, but this air may equally be introduced from the outer circumferential surface of the inhalation article 100.

At least part of the vibration generating unit 225 is preferably positioned between the first air flow path A1 and the inner wall surface of the accommodating portion 220. In this case, the vibration generating unit 225 is configured to be positioned between the first air flow path A1 and the second air flow path A2 when the inhalation article 100 is accommodated in the accommodating portion 220. By virtue of such a configuration, vibration can be efficiently transmitted to the consumable material 10, while the agglomerated powder 11 can also be dispersed, and it is also possible to improve conductance by moving obstructions by means of vibration if the first air flow path A1 is at least partially blocked.

In the example depicted, the bottom portion 223 is a movable member, and is configured to be movable along the direction of insertion of the inhalation article 100. An elastic member 240 such as a spring is arranged on the opposite side of the bottom portion 223 to the accommodating portion 220. The bottom portion 223 is connected to the elastic member 240, and is configured to bias the inhalation article 100 to the insertion end 201 side after the consumable material 10 has been pierced.

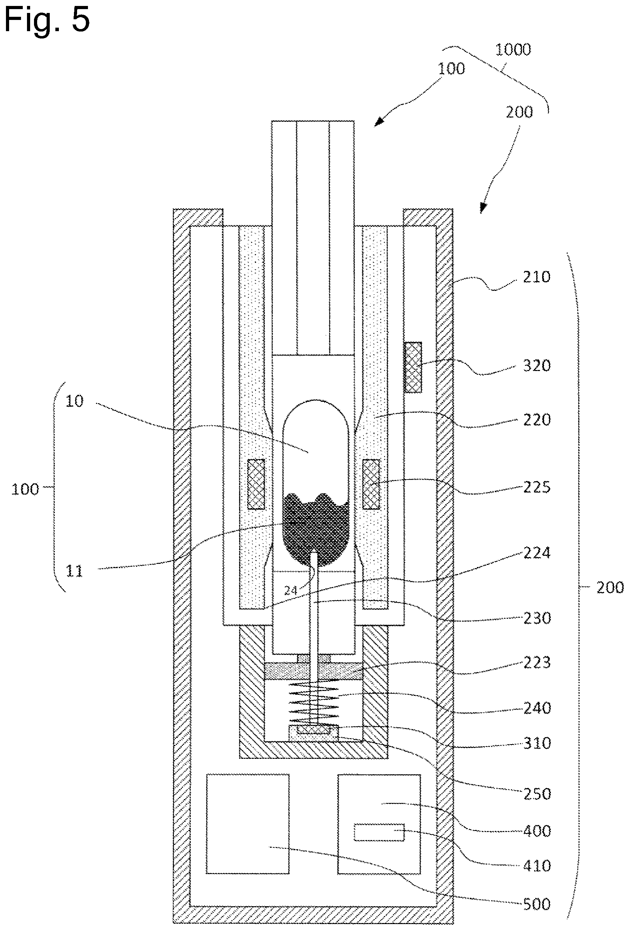

Fig. 5 is a view in cross section schematically showing the inhalation system 1000 immediately after the surface of the consumable material 10 has been pierced by the piercing portion 230. When the user inserts the inhalation article 100 into the accommodating portion 220, the first end 101 of the inhalation article 100 pushes the bottom portion 223 in while the consumable material 10 contacts the piercing portion 230 and a hole 24 is formed in the surface of the consumable material 10. Here, the powder 11 does not readily exit the consumable material 10 while the piercing portion 230 remains in the hole 24. The elastic member 240 is then configured so that the elastic member 240 compressed by pushing-in of the bottom portion 223 biases the bottom portion 223 and the inhalation article 100 to the insertion end 201 side, and the piercing portion 230 is readily detached from the consumable material 10. The elastic member 240 is preferably configured to bias the consumable material 10 to a position at which the vibration of the vibration generating unit 225 is readily transmitted to the consumable material 10. When the consumable material 10 separates from the piercing portion 230, the powder 11 is released to outside of the consumable material 10, and is released to outside of the inhalation article 100 through the air flow path A2. It should be noted that the bottom portion 223 need not be movable, and the inhalation device 200 need not comprise the elastic member 240. In this case, the piercing portion 230 may be configured to move in relation to the accommodating portion 220 in order to form a hole.

The piercing portion 230 comprises a piercing member for forming the hole 24 in the surface of the consumable material 10. This piercing member may be a rod-shaped or needle-shaped member extending in the longitudinal direction of the piercing portion 230, and may be configured so that the tip end on the insertion end 201 side penetrates the surface of the consumable material 10. The longitudinal direction of the piercing portion 230 preferably substantially coincides with the longitudinal direction of the inhalation device 200, but this is not limiting.

The inhalation device 200 comprises a detection unit 310 for enabling detection of whether or not the hole 24 has been correctly formed in the consumable material 10 by the piercing portion 230. Here, the hole 24 being "correctly" formed in the consumable material 10 means that the hole 24 is formed in a predetermined manner which may be defined by an algorithm, etc. in a control unit 400, as appropriate. For example, the detection unit 310 may be capable of detecting whether or not the hole 24 has been formed at the correct position in the consumable material 10, or may be capable of detecting whether or not the hole 24 has been formed in the consumable material weakened portion 23. If the hole 24 has not been correctly formed in the surface of the consumable material 10, the detection by the detection unit 310 allows the user or the inhalation device 200 to respond to this situation.

In this embodiment, the detection unit 310 is configured to detect a pressure applied to the piercing portion 230. This makes it possible to obtain information relating to what nature of contact the piercing portion 230 has made with the inhalation article 100, etc., and makes it possible to detect with greater certainty whether or not the hole 24 has been correctly formed in the consumable material 10. In the example of fig. 4, the detection unit 310 is a pressure sensor PS. The pressure sensor PS comprises a measurement face 311 and detects the pressure applied to the measurement face 311. A signal obtained by means of this detection is subjected to analog/digital (A/D) conversion, and is stored in a memory unit 410, etc. as pressure data. Since the detection unit 310 is the pressure sensor PS, it is possible to detect more accurately whether or not the hole 24 has been correctly formed in the consumable material 10, by utilizing the measured pressure data.

The pressure sensor PS is disposed on the opposite side along the longitudinal direction of the piercing portion 230 to the side on which the consumable material 10 is arranged. The measurement face 311 of the pressure sensor PS is configured to face the end portion of the piercing portion 230 on the opposite side to the end portion on the side which contacts the consumable material 10. By virtue of such a configuration, a force in the movement direction when the piercing portion 230 and the inhalation article 100 come closer together can be easily detected, and it is possible to detect even more accurately whether or not the hole 24 has been correctly formed in the consumable material 10. The inhalation device 200 has a support member 250 for supporting the pressure sensor PS. In the example depicted, the support member 250 is plate-shaped, but there is no particular limitation to the form thereof, provided that it can support the pressure sensor PS.

As shown in fig. 4, the inhalation device 200 further comprises the control unit 400 and a power source unit 500. The control unit 400 comprises a control device or a control circuit. The control unit 400 comprises a processor and a storage medium such as a memory, and controls operation of the inhalation device 200.

The control unit 400 determines whether or not the hole 24 has been correctly formed in the consumable material 10 by the piercing portion 230. This determination will be referred to below as a "piercing determination". By this means, it is possible to detect more accurately whether or not the hole 24 has been correctly formed in the consumable material 10, by analyzing, etc. the pressure data. The control unit 400 comprises the memory unit 410 which includes a non-volatile storage medium. The memory unit 410 stores a pressure value from when the piercing portion formed a hole in the surface of the consumable material, based on data, etc. obtained in the past in regard to piercing of the consumable material. This pressure value is referred to as a reference pressure value. The control unit 400 may make the piercing determination on the basis of the reference pressure value and output from the pressure sensor PS. This makes it possible to detect more accurately whether or not the hole 24 has been correctly formed in the consumable material 10, by utilizing data, etc. obtained in the past. For example, the control unit 400 calculates a similarity between the reference pressure value and the pressure in the pressure data, and may determine that the hole 24 has been correctly formed if the similarity is equal to or greater than a threshold, and may determine that the hole 24 has not been correctly formed if the similarity is less than the threshold. This similarity may be set on the basis of the difference between the reference pressure value and the pressure in the pressure data, or may be deduced by calculating respective feature values for the data and comparing the feature values. Alternatively, provided that the piercing determination can be made with the desired reliability, the piercing determination may be made by comparing the maximum value of the reference pressure value at the time of insertion of the inhalation article 100, and the maximum value of the pressure in the pressure data. It should be noted that there is no particular limitation as to the algorithm for making the piercing determination from the pressure data.

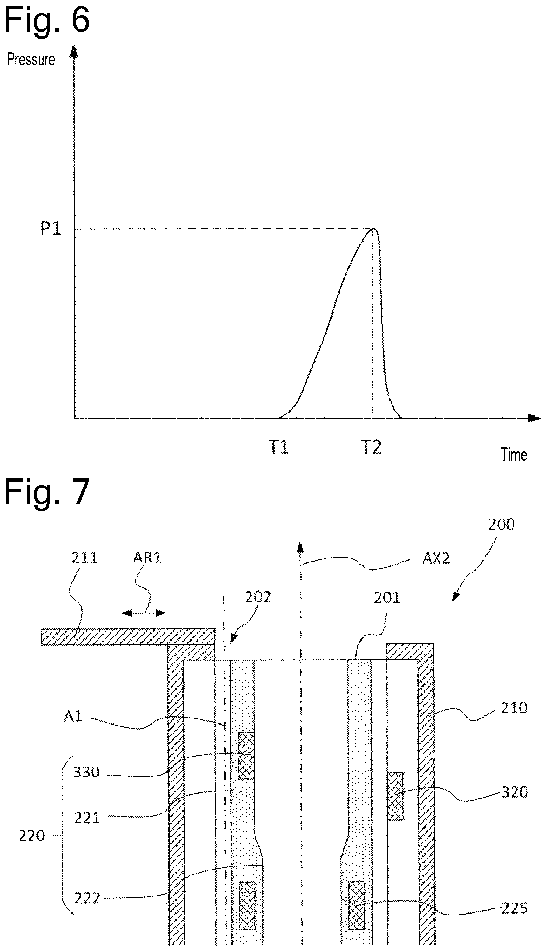

Fig. 6 is a graph showing an example of a profile of pressure applied to the pressure sensor PS at the time of insertion of the inhalation article 100. This graph shows time on the horizontal axis and pressure on the vertical axis. The piercing portion 230 contacts the surface of the consumable material 10 at a time T1. The force with which the consumable material 10 presses the piercing portion 230 increases while the piercing portion 230 is in contact with the surface of the consumable material 10 but the hole 24 has not been formed. When the hole 24 is formed at a time T2 at which the pressure assumes a maximum value P1, this pressure may sharply drop. It should be noted that this graph is merely an example depicted schematically, and the present invention is not limited to the content of the graph.

The control unit 400 may be configured to analyze input from the pressure sensor PS in real-time. In this case, the control unit 400 may be configured to cause the storage medium such as the memory unit 410 to store the pressure data if it has been detected by means of input from the pressure sensor PS that the pressure is rising. Furthermore, the control unit 400 may be configured to continue measurement by means of the pressure sensor PS when the pressure measured by the pressure sensor PS is rising, since the consumable material 10 is in contact with the piercing portion 230 but has not been pierced. The control unit 400 may be configured to stop measurement by the pressure sensor PS when the pressure measured by the pressure sensor PS is not rising, on the assumption that the piercing portion 230 has penetrated the surface of the consumable material 10 or that the piercing portion 230 has moved away from this surface. Alternatively, the control unit 400 may be configured to cause the storage medium such as the memory unit 410 to store the pressure data when the pressure measured by the pressure sensor PS is rising, since the consumable material 10 is in contact with the piercing portion 230 but has not been pierced. The control unit 400 may be configured to stop causing the pressure data to be stored when the pressure measured by the pressure sensor PS is not rising, on the assumption that the piercing portion 230 has penetrated the surface of the consumable material 10 or that the piercing portion 230 has moved away from this surface.

The control unit 400 may thus be configured to determine, based on whether or not the pressure obtained from the pressure sensor PS is rising, at least one of whether or not to continue measurement by the pressure sensor PS, and whether or not to record the measured pressure. This makes it possible to reduce power consumption and to cause the memory unit 410 to store the pressure data efficiently. As an example, the control unit 400 may start recording the pressure data from when the pressure is 0 or close to 0, at or close to the time T1, and may stop recording the pressure data or stop measurement of the pressure by the pressure sensor PS when the time T2, at which the pressure assumes the maximum value P1, is reached.

The control unit 400 may notify the user of information relating to the piercing determination, based on a result of the piercing determination. For example, when it is determined that the hole 24 has been correctly formed in the consumable material 10, the control unit 400 may notify the user that the hole 24 has been correctly formed by causing the vibration generating unit 225 to vibrate in a predetermined pattern. Alternatively or additionally, the control unit 400 may notify the user by controlling a sound source or a light source (not depicted) to emit sound or light. As an alternative, the control unit 400 may provide a notification that the hole 24 has been correctly formed by displaying characters, symbols, text or images, etc. on a display screen which is not depicted. The control unit 400 is thus preferably configured to notify the user of information relating to the piercing determination by means of at least one of sound, light, a screen, and vibration. This allows information relating to the piercing determination to be transmitted to the user more reliably.

As shown in fig. 4, the inhalation device 200 preferably comprises a shutter 211. The shutter 211 is configured to be capable of opening/closing the ventilation port 202. In the example depicted, the shutter 211 is also configured to be capable of opening/closing the opening formed at the insertion end 201 of the accommodating portion 220, but this is not limiting. In fig. 4, the arrows AR1 schematically illustrate the fact that the shutter 211 is formed to be slidable along the face of the inhalation device 200 where the insertion end 201 is disposed. There is no particular limitation as to the form of the shutter 211, provided that it is configured to be capable of opening/closing the ventilation port 202, etc., and the shutter 211 may equally be configured to be rotatable.

Opening/closing of the shutter 211 is preferably configured to be performed manually by the user. In this case, the inhalation device 200 preferably comprises a sensor (not depicted) for detecting opening/closing of the shutter 211. This sensor is configured to send signals or data relating to this detection to the control unit 400. For example, the control unit 400 may be configured so that the pressure sensor PS starts measuring the pressure when it is detected that the shutter 211 has moved from a closed position closing the ventilation port 202 to an open position opening the ventilation port 202. The control unit 400 may further be configured to stop measurement of the pressure by the pressure sensor PS when it is detected that the shutter 211 has moved from the open position to the closed position. The control unit 400 may thus be configured to switch the pressure sensor PS between a detection-possible state and a detection-impossible state according to the position of the shutter 211 disposed at the ventilation port 202 for introducing air into the accommodating portion 220. This makes it possible to stop measurement by the pressure sensor PS when it is unlikely that the inhalation article 100 is inserted in the accommodating portion 220, and it is possible to reduce power consumption. Moreover, it is also possible to adopt a configuration in which the pressure sensor PS starts measurement when pressure has been applied to the pressure sensor PS.

The control unit 400 may switch between an inhalation-possible state and an inhalation-impossible state of the inhalation device 200, based on the piercing determination, instead of or as well as providing the abovementioned notification relating to the piercing determination. This makes it possible to prevent the user from trying to inhale when the hole 24 has not been correctly formed by the piercing portion 230, but this leading to adverse effects such as the user being unable to inhale suitably, or the inhalation device 200 being damaged. The inhalation-possible state and the inhalation-impossible state of the inhalation device 200 may be switched by the control unit 400 controlling opening/closing of a solenoid valve (not depicted) arranged in the first air flow path A1. Alternatively, when opening/closing of the shutter 211 is controllable by the control unit 400, the abovementioned switch may be made by opening/closing the shutter 211. The control unit 400 may thus be configured to make the abovementioned switch by controlling opening/closing of the first air flow path A1 which introduces air into the accommodating portion 220. This makes it possible to more reliably prevent the user from trying to inhale when the hole 24 has not been correctly formed by the piercing portion 230, but this leading to adverse effects such as the user being unable to inhale suitably, or the inhalation device 200 being damaged.

The control unit 400 is configured to control vibration of the vibration generating unit 225. For example, the control unit 400 may switch the power supply to the vibration generating unit 225 on/off, based on a predetermined condition. Alternatively, the control unit 400 may be configured to switch the power supply on/off in multiple different patterns. By virtue of such a configuration, it is possible to disperse the agglomerated powder 11 more efficiently by means of the vibration generating unit 225, and it is possible to provide the user with information by means of the pattern of vibration of the vibration generating unit 225.

The inhalation device 200 preferably further comprises an inhalation sensor 320 for detecting inhalation by the user. A signal obtained by means of detection by the inhalation sensor 320 is A/D converted and transmitted to the control unit 400. The inhalation sensor 320 is preferably a pressure sensor. The control unit 400 is preferably configured to cause the vibration generating unit 225 to vibrate when inhalation by the user is detected due to a change in pressure. This makes it possible to disperse the agglomerated powder 11 when the user inhales and in preparation for the next inhalation, making it possible to more reliably suppress a drop in the efficiency of releasing the powder 11 by the inhalation device 200. Furthermore, it is also possible to inhibit the supply of power to the vibration generating unit 225 when the user is not inhaling, which can limit power consumption.

As shown in fig. 4, the inhalation sensor 320 is preferably arranged in the first air flow path A1. The inhalation sensor 320 is preferably configured to detect the pressure in the first air flow path A1. This makes the inhalation sensor 320 less likely to be contaminated with contents, etc. which detach from the inhalation article 100, as compared to when the inhalation sensor 320 is arranged in the accommodating portion 220, etc.

The control unit 400 may cause the memory unit 410, etc. to store the number of times that user inhalation has been detected. For example, the memory unit 410 may store a numerical value indicating a detection count, with the numerical value being set 0 when the power source of the inhalation device 200 is turned on, and 1 being added each time user inhalation is detected. A threshold may be prestored in the storage medium such as the memory unit 410, and the control unit 400 may vary the pattern of vibration of the vibration generating unit 225 when the detection count reaches or exceeds this threshold. This threshold will be referred to below as the "detection count threshold". Changes in the pattern of vibration may comprise changing the length of time of continuous vibration, and changing the length of at least one of periods of vibration and periods of interruption when there is intermittent vibration. Alternatively, the intensity of vibration may be varied, and this kind of change is also included in "changes in the pattern of vibration". The detection count threshold may be preset on the basis of the number of times that inhalation of the inhalation article 100 is possible, this number of times being obtained from past examples or a theoretical value. The control unit 400 is thus preferably configured to vary the pattern of vibration of the vibration generating unit 225 based on the number of times that user inhalation has been detected. This allows the user to be notified of changes in the number of times of inhalation, such as that the inhalation article 100 is at the end of use, or that the inhalation article 100 is nearing the end of use.

As shown in fig. 7, the inhalation device 200 may comprise an insertion sensor 330 for detecting insertion of the inhalation article 100. Fig. 7 is a view in cross section of the inhalation device 200 schematically showing the insertion sensor 330. The insertion sensor 330 may be an infrared sensor for detecting infrared radiation from the inhalation article 100. There is no particular limitation as to the type of insertion sensor 330, provided that it can detect insertion of the inhalation article 100, and it may equally be a pressure sensor. When the insertion sensor 330 is a pressure sensor, it is arranged on the inner wall surface of the accommodating portion 220 and detects contact with the inhalation article 100 that has been inserted into the accommodating portion 220. When the insertion sensor 330 is a pressure sensor, it is preferably arranged on the gripping portion 222.

The control unit 400 is preferably configured to cause vibration of the vibration generating unit 225 when the insertion sensor 330 detects insertion of the inhalation article 100 and the accommodating portion 220. This makes it possible to disperse the agglomerated powder 11 before the initial user inhalation, and makes it possible to inhibit a drop in the efficiency of releasing the powder 11 by the inhalation device 200 from the initial inhalation.

Returning to fig. 4, the power source unit 500 stores electrical power used by the inhalation device 200. The power source unit 500 is a battery such as a lithium ion battery, for example. The power source unit 500 may be rechargeable by means of an external power source, or may be non-rechargeable. The power source unit 500 may be a button battery from the perspective of making the power source unit 500 compact. The power source unit 500 supplies power to the vibration generating unit 225 and the detection unit 310, etc., as well as to the control unit 400. It should be noted that the power source unit 500 may comprise an element for performing piezoelectric conversion, and an element which generates electricity as a result of a temperature change based on the Seebeck effect, or the like. This allows the environment to be utilized to generate electricity even without a charger.

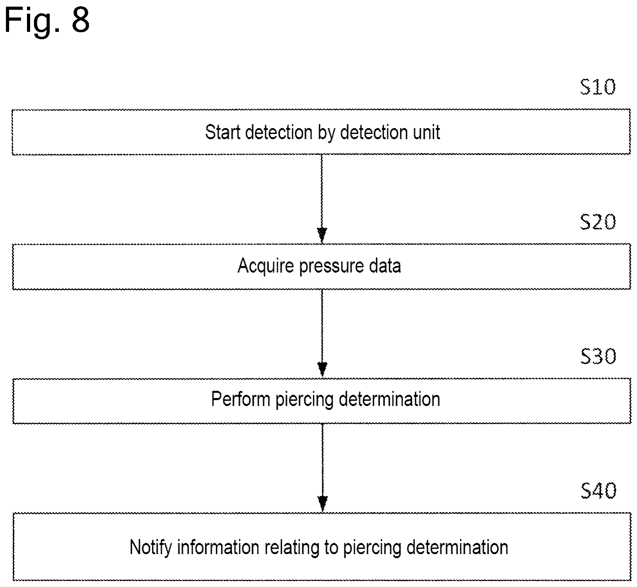

Fig. 8 is a flowchart showing the flow of control by the control unit 400 in this embodiment. Fig. 8 shows an example of the flow, and the present invention is not limited to this example. In step S10, when the power source of the inhalation device 200 is turned on, the control unit 400 controls the detection unit 310 to start detection by the pressure sensor PS. Step S20 is performed after step S10. In step S20, the control unit 400 acquires pressure data obtained by detection from the detection unit 310. Step S30 is performed after step S20. In step S30, the control unit 400 performs the piercing determination to determine whether or not the hole 24 has been correctly formed. Step S40 is performed after step S30. In step S40, the control unit 400 controls the vibration generating unit 225, etc. in order to provide a notification of information relating to the piercing determination, such as whether or not the hole 24 has been correctly formed.

The inhalation device 200 and the inhalation system 1000 of this embodiment comprise: the housing 210, the accommodating portion 220 enabling the consumable material 10 to be accommodated inside the housing 210, the piercing portion 230 for forming the hole 24 in the surface of the consumable material 10; and the detection unit 310 configured to detect the pressure applied to the piercing portion 230. This enables the inhalation device 200 to reliably detect that the hole 24 has not been correctly formed in the surface of the consumable material 10 by the piercing portion 230, thus allowing the user or the inhalation device 200 to respond to this situation.

A powder inhaler (inhalation device 200) and a powder inhalation system (inhalation system 1000) of this embodiment comprise: the accommodating portion 220 for accommodating the inhalation article 100 which contains the powder 11 for inhalation; and one or more vibration generating units 225 configured to be capable of vibrating the accommodating portion 220. This makes it possible to provide a powder inhaler and a powder inhalation system having a novel configuration capable of inhibiting a drop in the efficiency of releasing the powder 11, even if the powder 11 has agglomerated in the inhalation article 100.

### Second embodiment

An inhalation system 1000A of the second embodiment is partially the same as the inhalation system 1000 of the first embodiment in terms of configuration. However, the inhalation system 1000A differs from the inhalation system 1000 in that the inhalation system 1000A comprises an inhalation device 200A instead of the abovementioned inhalation device 200. The inhalation device 200A differs from the inhalation device 200 according to the first embodiment in that the inhalation device 200A comprises a detection unit 310A instead of the detection unit 310, and further comprises a window portion 212. The inhalation device 200A also differs from the inhalation device 200 in that the inhalation device 200A does not comprise the power source unit 500 and does not have any electrically operated parts. Parts which are the same as those of the first embodiment are referenced by the same reference signs as in the first embodiment and a description thereof will be omitted where appropriate.

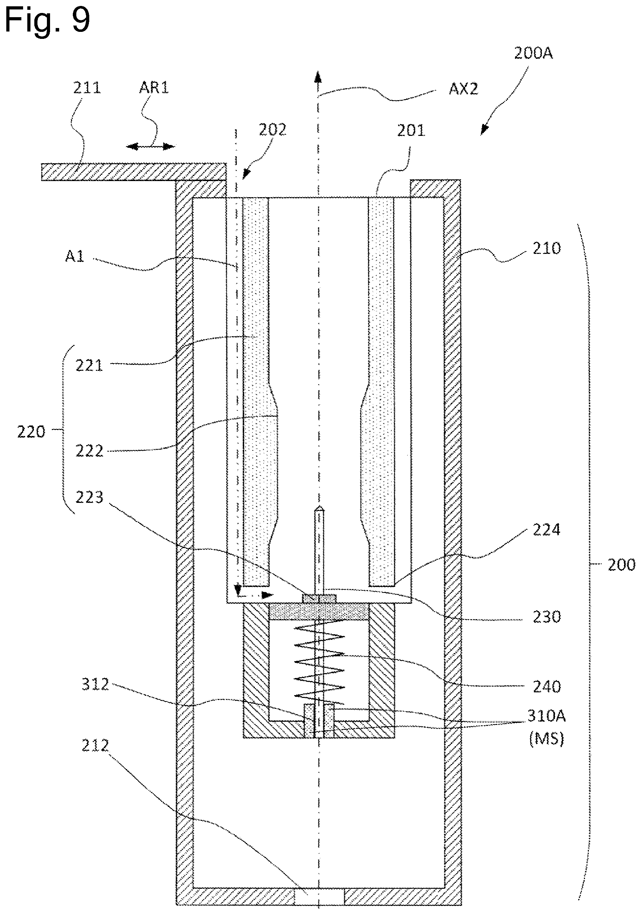

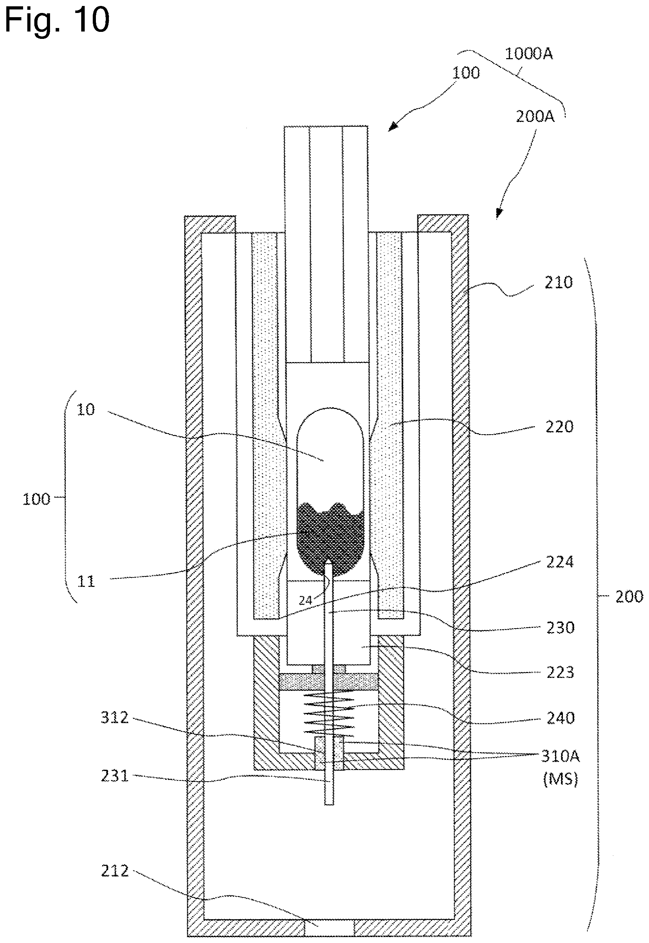

Fig. 9 is a view in cross section schematically showing the inhalation device 200A according to the second embodiment. Fig. 10 is a view in cross section schematically showing the inhalation system 1000A of this embodiment with the inhalation article 100 inserted in the accommodating portion 220. In this embodiment, the detection unit 310A varies a part of the inhalation device 200A which is visible, according to whether or not the hole 24 has been correctly formed by the piercing portion 230. By this means, the detection unit 310A enables the user, etc. to detect whether or not the hole 24 has been correctly formed by the piercing portion 230.

As shown in fig. 9, a through-hole 312 is formed in the detection unit 310A. The through-hole 312 extends in the insertion direction of the inhalation article 100 and is configured to allow penetration by the piercing portion 230. The detection unit 310A is configured to contact and support the piercing portion 230 which is inside the through-hole 312 when the inhalation article 100 is not inserted in the accommodating portion 220. As shown in fig. 10, the piercing portion 230 comprises a base end portion 231 which is on the opposite side to the end portion in contact with the consumable material 10. The detection unit 310A is configured so that, when the inhalation article 100 is inserted into the accommodating portion 220, and the consumable material 10 is contacted by the piercing portion 230 and applies a force in the insertion direction, the piercing portion 230 is moved by this force and moves to a position where the base end portion 231 is visible from the window portion 212. Moreover, the piercing portion 230 may be configured to be biased to the consumable material 10 side by means of an elastic body such as a spring.

The base end portion 231 moves to roughly the same position when the hole 24 has been correctly formed in the consumable material 10. Meanwhile, if the hole 24 has not been correctly formed in the consumable material 10, the base end portion 231 moves to a different position from when the hole 24 has been correctly formed. For example, if the hole 24 is correctly formed, the hole 24 is formed before the consumable material 10 reaches its position furthest to the piercing portion 230 side. Meanwhile, if the hole 24 is not correctly formed, the distance of longitudinal movement of the piercing portion 230 may become longer because the piercing portion 230 is pushed in until the consumable material 10 reaches its position furthest to the piercing portion 230 side. The length of the base end portion 231 exposed at a position where it is visible to the user therefore differs according to whether or not the hole 24 is correctly formed. This allows the user, etc. to detect whether or not the hole 24 has been correctly formed. It should be noted that the window portion 212 need not be formed, provided that the user, etc. is able to see through, and an opening portion may equally be formed in the housing 210 instead of the window portion 212.

The detection unit 310A is preferably a mechanical structure MS which enables detection of whether or not the hole 24 is correctly formed, without the need for electrical power. This makes it possible to construct the inhalation device 200A and the inhalation system 1000A with a simple configuration that does not require electricity. Furthermore, the inhalation system 1000A may comprise the control unit 400, power source unit 500, and sensors, etc. which are identical to those of the inhalation device 200 of the first embodiment, in which case it is possible to reduce the power needed to detect whether or not the hole 24 is correctly formed. It should be noted that the detection unit 310A may be formed with a weakened portion which obstructs the through-hole 312, and may be configured so that the weakened portion is deformed or pushed out by the piercing portion 230 when the inhalation article 100 is inserted into the accommodating portion 220, whereby the through-hole 312 is formed.

In this embodiment, the detection unit 310A is a mechanical structure MS which is configured so that when the consumable material 10 has been inserted into the accommodating portion 220 but the hole 24 has not been formed by the piercing portion 230, part of the piercing portion 230 is pushed out, through the weakened portion or the through-hole 312 formed in the inner wall of the accommodating portion 220, to a position where it is visible from outside of the inhalation device 200A. As a result, when the hole 24 has not been correctly formed in the surface of the consumable material 10, it is possible to detect that the hole has not been correctly formed, without electrical power necessarily being required, therefore enabling a user, etc. to respond to the situation.

### Third embodiment

An inhalation system 1000B of a third embodiment has substantially the same configuration as that of the inhalation system 1000 of the first embodiment. However, the inhalation system 1000B differs from the inhalation system 1000 in that the inhalation system 1000A comprises an inhalation device 200B instead of the abovementioned inhalation device 200. The inhalation device 200B differs from the inhalation device 200 of the first embodiment in that the inhalation device 200B comprises a piercing portion 230A and a detection unit 310B instead of the piercing portion 230 and the detection unit 310. Parts which are the same as those of the first embodiment are referenced by the same reference signs as in the first embodiment and a description thereof will be omitted where appropriate.

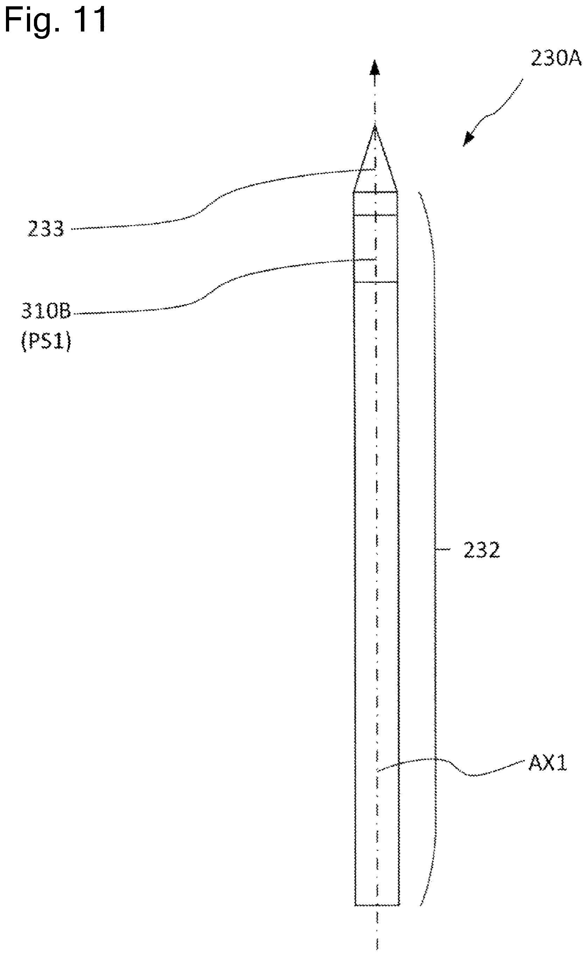

Fig. 11 is a side view schematically showing the piercing portion 230A according to this embodiment. The piercing portion 230A is formed by a needle-shaped member extending in the longitudinal direction along the long axis AX1. The piercing portion 230A comprises a piercing portion main body 232 and a tip end portion 233. The piercing portion main body 232 is formed with a columnar shape extending in the longitudinal direction. The tip end portion 233 is configured to contact the consumable material 10. In the example depicted, the tip end portion 233 is formed to become narrower toward the tip end of the piercing portion 230A on the consumable material 10 side, but this is not limiting provided that the consumable material 10 can be penetrated, and the piercing portion 230A may be rod-shaped rather than needle-shaped. The detection unit 310B is a pressure sensor PS1. In the example depicted, the detection unit 310B is arranged on the outer circumferential surface of the piercing portion main body 232, but this is not limiting provided that it is capable of detecting pressure in a direction intersecting the longitudinal direction, and the detection unit 310B may equally be provided on the tip end portion 233.

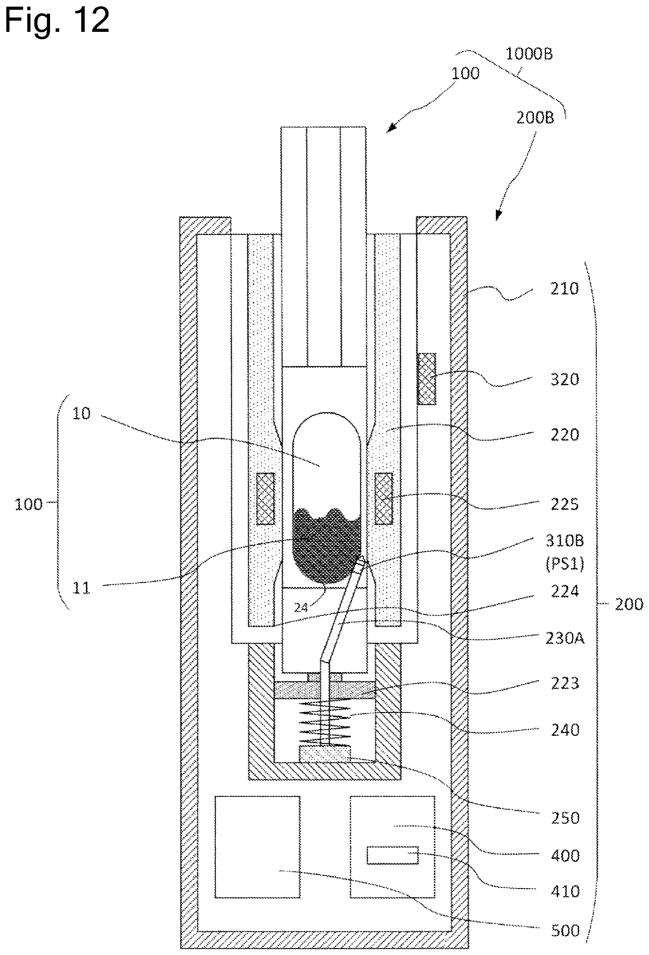

Fig. 12 is a view in cross section of the inhalation system 1000B, schematically showing detection by the detection unit 310B of whether or not the hole 24 has been correctly formed. When the inhalation article 100 is inserted into the accommodating portion 220, there is a possibility that the piercing portion 230A will break or flex toward the inner wall surface side constituting the accommodating portion 220, without penetrating the consumable material 10. According to this embodiment, in such a case, a wrapper, etc. of the consumable material 10 or the inhalation article 100 contacts the detection unit 310B, and the detection unit 310B detects this contact to thereby make it possible to detect that the hole 24 has not been correctly formed. The detection unit 310B may be configured to detect at least one of bending and flexing of the piercing portion 230A. For example, if the detection unit 310B has detected a pressure equal to or greater than a predetermined threshold, the control unit 400 may determine that pressure has been applied to the piercing portion 230A from a direction other than the longitudinal direction, and may determine that the hole 24 has not been correctly formed.

In this embodiment, the piercing portion 230A is a needle-shaped or rod-shaped member extending in the longitudinal direction, and the detection unit 310B is configured to detect a pressure on the piercing portion 230A in a direction intersecting the longitudinal direction. By this means, the inhalation device 200B can detect, etc. bending or flexing of the piercing portion 230A, and can detect more reliably whether or not the hole 24 has been correctly formed.

Embodiments of the present invention were described above, but the present invention is not limited to those embodiments, and various modifications are possible within the scope of the technical concept disclosed in the claims, specification and drawings. Moreover, any shape or material not directly stated in the specification or drawings is also within the scope of the technical concept of the invention of this application, provided that it exhibits the action and effect of the invention of this application.

According to a first aspect of the present invention, the inhalation device comprises: a housing; an accommodating portion enabling a consumable material to be accommodated inside the housing; a piercing portion for forming a hole in a surface of the consumable material; and a detection unit for enabling detection of whether or not the hole has been correctly formed by the piercing portion.
According to a second aspect of the present invention, which is in accordance with the first aspect, the detection unit is configured to detect a pressure applied to the piercing portion.
According to a third aspect of the present invention, which is in accordance with the second aspect, the detection unit is a pressure sensor.
According to a fourth aspect of the present invention, which is in accordance with the third aspect, the piercing portion is a rod-shaped or needle-shaped member extending in a longitudinal direction, and the pressure sensor is disposed on the opposite side along the longitudinal direction to the side on which the consumable material is arranged.
According to a fifth aspect of the present invention, which is in accordance with the third or fourth aspect, a control unit is further provided to determine whether or not the hole has been correctly formed in the consumable material by the piercing portion. According to a sixth aspect of the present invention, which is in accordance with the fifth aspect, the control unit comprises a memory unit for storing a pressure value from when the piercing portion formed a hole in the surface of the consumable material, and makes the abovementioned determination based on the pressure value and output from the pressure sensor.
According to a seventh aspect of the present invention, which is in accordance with the fifth or sixth aspect, based on whether or not the pressure obtained from the pressure sensor is rising, the control unit determines at least one of: whether or not to continue measurement by the pressure sensor, and whether or not to record the measured pressure.
According to an eighth aspect of the present invention, which is in accordance with the fifth aspect, the piercing portion is a rod-shaped or needle-shaped member extending in a longitudinal direction, and the pressure sensor is configured to detect a pressure on the piercing portion in a direction intersecting the longitudinal direction.
According to a ninth aspect of the present invention, which is in accordance with any of the fifth to eighth aspects, the control unit is configured to notify a user of information relating to the abovementioned determination by means of at least one of sound, light, a screen, and vibration.
According to a 10th aspect of the present invention, which is in accordance with any of the fifth to ninth aspects, based on the abovementioned determination, the control unit is configured to switch between an inhalation-possible state and an inhalation-impossible state of the inhalation device.
According to an 11th aspect of the present invention, which is in accordance with the 10th aspect, the control unit is configured to perform the abovementioned switch by controlling opening/closing of a flow path for introducing air into the accommodating portion.
According to a 12th aspect of the present invention, which is in accordance with any of the fifth to 11th aspects, the control unit is configured to switch the pressure sensor between a detection-possible state and a detection-impossible state according to a position of a shutter disposed at an introduction port for introducing air into the accommodating portion.
According to a 13th aspect of the present invention, which is in accordance with the first aspect, the detection unit is a mechanical structure which is configured so that, when the consumable material has been inserted into the accommodating portion but the hole has not been formed by the piercing portion, a portion of the piercing portion is pushed out, to a position where it is visible from outside of the inhalation device, through a weakened portion or a through-hole formed in an inner wall of the accommodating portion.
According to a 14th aspect of the present invention, which is in accordance with any of the first to 13th aspects, the consumable material comprises a flavor powder, and the inhalation device is a flavor inhaler for inhaling a flavor.
According to a 15th aspect of the present invention, an inhalation system comprises: the inhalation device according to any of the first to 14th aspects; and a consumable material which can be accommodated in the inhalation device.

### REFERENCE SIGNS LIST

10 Consumable material
11 Powder
12 Particle
23 Consumable material weakened portion
24 Hole
100 Inhalation article
120 Powder-containing segment
200, 200A, 200B Inhalation device
201 Insertion end
202 Ventilation port
210 Housing
211 Shutter
212 Window portion
220 Accommodating portion
221 Tubular portion
222 Gripping portion
223 Bottom portion
224 Air introduction port
225 Vibration generating unit
230, 230A Piercing portion
231 Base end portion
310, 310A, 310B Detection unit
312 Through-hole
320 Inhalation sensor
330 Insertion sensor
400 Control unit
410 Memory unit
500 Power source unit
1000, 1000A, 1000B Inhalation system
A1 First air flow path
A2 Second air flow path
AX1 Long axis of inhalation article
AX2 Center axis of accommodating portion
AX10 Long axis of consumable material
MS Mechanical structure
PS, PS1 Pressure sensor

## Claims

1. An inhalation device comprising:
a housing;
an accommodating portion enabling a consumable material to be accommodated inside the housing;
a piercing portion for forming a hole in a surface of the consumable material; and
a detection unit for enabling detection of whether or not the hole has been correctly formed by the piercing portion.

2. The inhalation device as claimed in claim 1, wherein the detection unit is configured to detect a pressure applied to the piercing portion.

3. The inhalation device as claimed in claim 2, wherein the detection unit is a pressure sensor.

4. The inhalation device as claimed in claim 3, wherein the piercing portion is a rod-shaped or needle-shaped member extending in a longitudinal direction, and
the pressure sensor is disposed on the opposite side along the longitudinal direction to the side on which the consumable material is arranged.

5. The inhalation device as claimed in claim 3 or 4, further comprising a control unit for determining whether or not the hole has been correctly formed in the consumable material by the piercing portion.

6. The inhalation device as claimed in claim 5, wherein the control unit comprises a memory unit for storing a pressure value from when the piercing portion formed a hole in the surface of the consumable material, and makes the abovementioned determination based on the pressure value and output from the pressure sensor.

7. The inhalation device as claimed in claim 5 or 6, wherein, based on whether or not the pressure obtained from the pressure sensor is rising, the control unit determines at least one of: whether or not to continue measurement by the pressure sensor, and whether or not to record the measured pressure.

8. The inhalation device as claimed in claim 5, wherein the piercing portion is a rod-shaped or needle-shaped member extending in a longitudinal direction, and
the pressure sensor is configured to detect a pressure on the piercing portion in a direction intersecting the longitudinal direction.

9. The inhalation device as claimed in any one of claims 5 to 8, wherein the control unit is configured to notify a user of information relating to the abovementioned determination by means of at least one of sound, light, a screen, and vibration.

10. The inhalation device as claimed in any one of claims 5 to 9, wherein, based on the abovementioned determination, the control unit is configured to switch between an inhalation-possible state and an inhalation-impossible state of the inhalation device.

11. The inhalation device as claimed in claim 10, wherein the control unit is configured to perform the abovementioned switch by controlling opening/closing of a flow path for introducing air into the accommodating portion.

12. The inhalation device as claimed in any one of claims 5 to 11, wherein the control unit is configured to switch the pressure sensor between a detection-possible state and a detection-impossible state according to a position of a shutter disposed at an introduction port for introducing air into the accommodating portion.

13. The inhalation device as claimed in claim 1, wherein the detection unit is a mechanical structure which is configured so that, when the consumable material has been inserted into the accommodating portion but the hole has not been formed by the piercing portion, a portion of the piercing portion is pushed out, to a position where it is visible from outside of the inhalation device, through a weakened portion or a through-hole formed in an inner wall of the accommodating portion.

14. The inhalation device as claimed in any one of claims 1 to 13, wherein the consumable material comprises a flavor powder, and the inhalation device is a flavor inhaler for inhaling a flavor.

15. An inhalation system comprising:
the inhalation device as claimed in any one of claims 1 to 14; and
a consumable material which can be accommodated in the inhalation device.
